# FASCICULE DE BREVET EUROPEEN

(11) **EP 4 547 629 B1**
(45) Date de publication et mention de la délivrance du brevet: **19.08.2026**
(21) Numéro de dépôt: 23735318.0
(22) Date de dépôt: 27.06.2023
(51) Int. Cl.: C07C 4/22, C08J 11/12, C10G 1/10

(54) **PROCÉDÉ DE DÉPOLYMÉRISATION D'UNE CHARGE POLYSTYRÉNIQUE PAR PYROLYSE**
VERFAHREN ZUR DEPOLYMERISATION EINES POLYSTYROLROHSTOFFES DURCH PYROLYSE
PROCESS FOR THE DEPOLYMERIZATION OF A POLYSTYRENE FEEDSTOCK BY PYROLYSIS

(30) Priorité: 30.06.2022 FR 2206627
(43) Date de publication de la demande: 07.05.2025
(73) Titulaire: COMPAGNIE GENERALE DES ETABLISSEMENTS MICHELIN, 63000 Clermont-Ferrand (FR); Pyrowave Inc., Oakville ON L6H OC3 (CA)
(72) Inventeur: MAISONNEUVE, Lise, 63040 CLERMONT-FERRAND CEDEX 09 (FR); MATHIEU, Florent, 63040 CLERMONT-FERRAND CEDEX 09 (FR)
(74) Mandataire: M.F.P. Michelin
(86) Numéro de dépôt international: PCT/EP2023/067414
(87) Numéro de publication internationale: WO 2024/003021

(56) Documents cités:
- WO-A1-2021/180893
- WO-A1-2022/180030
- US-A1- 2022 411 351

## Description

### Domaine technique de l'invention

La présente invention est relative au domaine des procédés de dépolymérisation des composés polystyrénique, en particulier du polystyrène, en vue de produire au moins un monomère styrène.

### Art antérieur

Le styrène est un monomère très largement utilisé dans l'industrie, que ce soit par exemple pour la production de polystyrène, dont les domaines d'application sont multiples, ou la production d'élastomères tels que le caoutchouc styrène-butadiène (SBR). Il peut être obtenu de multiples façons, la principale étant par la déshydrogénation de l'éthylbenzène, ou dans une moindre mesure par l'oxydation de l'éthylbenzène suivie de la réaction sur le propylène puis de la déshydratation du produit obtenu.

Dans une optique de réduction de pression sur les ressources fossiles, des développements récents ont porté sur la dépolymérisation des composés styréniques tels que le polystyrène. Un tel procédé est, par exemple, décrit dans la demande US 2021/0277202.

Lors de ces procédés, le polystyrène est décomposé à haute température dans un environnement anaérobie en composés de plus bas poids moléculaire, dont le styrène. Cependant, une quantité significative d'autres produits sont également générés, notamment des oligomères du styrène. Ces oligomères peuvent représenter jusqu'à 40% massique du polystyrène entrant et comprennent en majorité des dimères et des trimères de styrène, parmi lesquels notamment le 1,3-diphenylpropane, le 1,3-diphenylbutene-1, le 1,2-diphenylpropane, le 1,3-diphenylbutane et le 1,4-diphenylbenzene. La présence de ces oligomères peut s'expliquer d'une part par des réactions de dépolymérisation partielle du polystyrène en oligomères, et d'autre part par la polymérisation ou la recombinaison radicalaire du styrène ou d'oligomères dans le réacteur de dépolymérisation et/ou dans les flux sortant de ce réacteur. Le document US 2021/0277202 propose de vapocraquer les oligomères de styrène produits afin de générer des composés plus légers tels que éthène, propène ou benzène.

D'autres voies sont envisageables pour valoriser les oligomères de styrène et améliorer le rendement global en styrène de ces procédés. Le document US 10,731,080 propose de recycler ces oligomères dans la charge du réacteur de pyrolyse. Cette démarche de recyclage, qui est classiquement mise en œuvre dans les procédés pour lesquels la conversion en produit d'intérêt est incomplète, n'est cependant pas totalement satisfaisante en l'espèce.

En effet, à pression ambiante, les oligomères ont un point d'ébullition proche de la température de décomposition du polystyrène. Le recyclage des oligomères dans le réacteur de dépolymérisation affecte la chimie de la décomposition du polystyrène en réduisant la sélectivité et le rendement des monomères, car l'évaporation des oligomères sera en compétition avec la décomposition du polystyrène. L'évaporation des oligomères de styrène, en consommant une partie importante de l'énergie, réduit ainsi la quantité d'énergie disponible pour la décomposition du polystyrène, affectant par conséquent la température de réaction, le rendement de réaction et la sélectivité vers la production de monomère de styrène. Cette solution peut donc encore être améliorée.

Une autre solution pourrait consister, afin de contrer les effets présentés ci-dessus, à augmenter la pression opératoire de la section de pyrolyse afin d'augmenter la température de vaporisation des oligomères de styrène et le taux d'évaporation à la température à laquelle la pyrolyse est opérée. Cependant, cette augmentation de pression opératoire augmentera également la température de vaporisation du styrène, augmentant son temps de séjour dans la phase liquide et gazeuse où il se décomposera davantage en composés plus légers tels que des produits non condensables plus légers (hydrogène ainsi que des alcanes et alcènes en C1, C3, C4 et C5). Dans l'ensemble, l'augmentation de la pression du réacteur diminue le rendement en liquide produit, et le rendement en styrène.

Le document WO 2021/180893 propose un procédé dans lequel une charge polystyrénique est pyrolysée dans un premier réacteur opéré à une pression inférieure à 1 bar puis séparée en une fraction légère comprenant au moins une partie du monomère styrène et une fraction lourde comprenant au moins une partie des oligomères de styrène, cette fraction lourde étant traitée dans un deuxième réacteur de pyrolyse opéré dans des conditions différentes du premier réacteur de pyrolyse, notamment une pression supérieure à 1 bar et une température inférieure à 650°C. Cependant, le contrôle de la composition de la fraction lourde n'est pas abordé. Or celle-ci a une influence importante sur les performances du deuxième réacteur de pyrolyse.

Poursuivant ses recherches, la demanderesse a découvert un procédé de dépolymérisation d'une charge polystyrénique dans lequel la conversion de la charge polystyrénique en monomère styrène est améliorée par la mise en œuvre de deux étapes de pyrolyse et par le contrôle de l'opération de la seconde étape, et notamment par la régulation de la présence d'un agent donneur d'hydrogène.

### Description détaillée de l'invention

Ainsi, l'invention concerne un procédé de dépolymérisation d'une charge polystyrénique comprenant au moins les étapes suivantes :
a. Une étape de préparation de la charge polystyrénique afin de provoquer la fonte des composés plastiques ;
b. Une étape de première pyrolyse de la charge issue de l'étape a) comprenant une section de pyrolyse et produisant au moins un premier flux de pyrolyse gazeux et un premier flux de pyrolyse liquide ;
c. Une étape de séparation alimentée au moins par le premier flux de pyrolyse gazeux issu de l'étape b) et le deuxième flux de pyrolyse gazeux issu de l'étape d) et produisant au moins un flux riche en composés légers, un flux riche en oligomères, et un flux riche en aromatiques ;
d. Une étape de deuxième pyrolyse comprenant une section de pyrolyse alimentée au moins par le flux riche en oligomères issu de l'étape c), et produisant au moins un deuxième flux de pyrolyse gazeux et un deuxième flux de pyrolyse liquide ;
e. Une étape de séparation du flux riche en aromatiques issu de l'étape c) en au moins un flux comprenant majoritairement de l'éthylbenzène, un flux comprenant majoritairement du styrène et un flux de composés lourds ;
dans lequel on contrôle, dans la section de pyrolyse de l'étape d), la pression partielle en hydrogène et/ou le temps de séjour de la phase gaz.

### Définitions

Les composés comprenant du carbone mentionnés dans la description peuvent être d'origine fossile ou biosourcés. Dans ce dernier cas, ils peuvent être, partiellement ou totalement, issus de la biomasse ou obtenus à partir de matières premières renouvelables issues de la biomasse. Sont concernés notamment les polymères, les plastifiants, les charges, etc.

Tout intervalle de valeurs désigné par l'expression "entre a et b" représente le domaine de valeurs allant de plus de a à moins de b (c'est-à-dire bornes a et b exclues) tandis que tout intervalle de valeurs désigné par l'expression "de a à b" signifie le domaine de valeurs allant de a jusqu'à b (c'est-à-dire incluant les bornes strictes a et b).

Le terme « donneur d'hydrogène » désigne un composé pouvant réagir avec les oligomères du styrène lors de la réaction de pyrolyse en fournissant des atomes d'hydrogène. Un tel composé peut être, par exemple, une cire telle qu'une cire paraffinique, une polyoléfine thermoplastique telle que le polyéthylène ou le polypropylène, ou le dihydrogène.

Par « cire paraffinique », on entend de manière connue un alcane linéaire ou ramifié solide à température ambiante, dont la température de fusion est inférieure à 100°C. Une cire paraffinique comprend typiquement de 20 à 40 atomes de carbone.

Par "monomère styrène", on entend le composé styrène, de formule C₈H₈.

Par « composé majoritaire », on comprend que le composé est présent à plus de 50% en masse dans le flux considéré.

### Charge du procédé de dépolymérisation

Le procédé selon l'invention est un procédé de dépolymérisation d'une charge polystyrénique. Par charge polystyrénique, on entend une charge qui comprend des polymères à base de styrène, tels que les caoutchoucs styréniques et le polystyrène.

De préférence, la charge du procédé est une charge polystyrénique issue de déchets plastiques. Une telle charge comprend préférentiellement au moins 90% poids de polystyrène, de préférence au moins 93% poids de polystyrène, et de manière préférée au moins 95% poids de polystyrène. La charge polystyrénique peut comprendre d'autres composés, en particulier si elle est issue de déchets plastiques. Ces autres composés peuvent être, de manière non limitative, des composés plastiques tels que du polyéthylène, du polypropylène, des élastomères, des matériaux organiques tels que du papier, de la nourriture, ou inorganiques tels que du verre, du métal, du sable.

La charge polystyrénique du procédé selon l'invention peut contenir un donneur d'hydrogène au sens de la présente invention. Par exemple, dans le cas où le donneur d'hydrogène est le polypropylène, le ratio massique polypropylène sur polystyrène dans la charge polystyrénique peut être ajusté entre 1% poids et 15% poids, de préférence entre 1% et 3% poids.

### Étape a) de préparation

Le procédé selon l'invention comprend une étape de préparation de la charge polystyrénique. Lors de cette étape, la charge polystyrénique est conditionnée pour pouvoir alimenter l'étape b) de première pyrolyse. Cette étape de préparation peut comprendre des opérations de broyage, de dégazage, de mise en température afin de provoquer la fonte des composés plastiques par exemple dans un dispositif d'extrusion au cours duquel la température est graduellement augmentée. Les effluents vapeurs (eau, composés légers générés par la décomposition partielle de la charge polystyrénique) et les effluents solides (débris non fusibles tels que les débris métalliques, verre) sont séparés.

De préférence, la charge polystyrénique est progressivement chauffée à une température comprise entre 200°C et 300°C, cette température permettant d'obtenir la fusion du polystyrène en limitant sa décomposition thermique.

### Étape b) de première pyrolyse

Le procédé selon l'invention comprend une étape de première pyrolyse de la charge issue de l'étape a) comprenant une section de pyrolyse et produisant au moins un premier flux de pyrolyse gazeux et un premier flux de pyrolyse liquide.

Par pyrolyse, on entend la décomposition thermique de composés dans une atmosphère inerte.

La charge conditionnée issue de l'étape a), préférentiellement sous forme fondue, alimente une section de pyrolyse, opérée à une température et une pression telles que la dépolymérisation du polystyrène en oligomères de styrène et en monomère de styrène ait lieu. De préférence, la section de pyrolyse est opérée à une température allant de 300 à 700°C, de préférence allant de 300°C à 600°C. De préférence, la section de pyrolyse est opérée à une pression allant de 0,1 bar à 2 bar, préférentiellement allant de 0,5 bar à 1,5 bar et très préférentiellement de 0,8 bar à 1,2 bar. Dans ces conditions, la quantité d'oligomères de styrène dans le premier flux de pyrolyse gazeux peut aller jusqu'à 40% en masse par rapport à la quantité de polystyrène dans la charge de la section de pyrolyse.

De préférence, la section de pyrolyse met en œuvre une section de pyrolyse par micro-onde. Une telle pyrolyse par micro-onde utilisable pour la pyrolyse d'une charge polystyrénique est par exemple décrite dans le document WO 2020/202089.

L'utilisation d'une section de pyrolyse assistée par micro-ondes permet d'atteindre des taux de transfert de chaleur et des températures de réaction plus élevés, qui favorisent les réactions de scission en fin de chaîne et minimisent la formation d'oligomères de styrène. Une section de pyrolyse par micro-ondes se caractérise également par une température dans la masse réactionnelle inférieure à celle d'une section de pyrolyse conventionnelle. La baisse des températures dans la masse réactionnelle entraîne une baisse des taux d'évaporation des oligomères de styrène et évite de « sur-craquer » le styrène produit. L'utilisation d'une section de pyrolyse par micro-ondes réduira la formation d'oligomères de styrène par rapport à une section de pyrolyse conventionnelle, sans toutefois éviter totalement leur formation, qui peut encore représenter, dans le premier flux de pyrolyse gazeux, jusqu'à 40 % en masse par rapport à la quantité de polystyrène dans la charge de la section de pyrolyse.

La section de pyrolyse produit un premier flux de pyrolyse gazeux et un premier flux de pyrolyse liquide. Le premier flux de pyrolyse gazeux peut également contenir des gouttelettes de liquide entrainées. Outre des oligomères du styrène, le premier flux de pyrolyse gazeux comprend la majorité du monomère styrène produit dans la section de pyrolyse, ainsi que des composés aromatiques légers gazeux dans les conditions opératoires tels que alpha-méthyl-styrène, éthylbenzène, cumène et toluène.

Le premier flux de pyrolyse gazeux comprend majoritairement du styrène, c'est-à-dire au moins 50% en poids de styrène, préférentiellement au moins 60% en poids de styrène.

De manière préférée, le premier flux de pyrolyse gazeux comprend au plus 10% poids d'éthylbenzène, de préférence au plus 5% poids d'éthylbenzène et de manière préférée au plus 3% poids d'éthylbenzène.

Préférentiellement, le premier flux de pyrolyse gazeux comprend au moins 10% poids de composés dont le point d'ébullition est supérieur à celui du styrène.

Le premier flux de pyrolyse liquide peut également comprendre des éléments solides, polymères non fusionnés, produits lors de la pyrolyse, ou des débris non séparés dans l'étape de préparation de la charge. Ce flux alimente, de manière préférée, une section de séparation dans laquelle la fraction solide éventuelle est séparée de la fraction liquide, cette dernière pouvant être recyclée en mélange avec l'alimentation de la section de pyrolyse de l'étape de première pyrolyse.

### Étape c) de séparation

Le procédé selon l'invention comprend une étape de séparation alimentée au moins par le premier flux de pyrolyse gazeux issu de l'étape b) et le deuxième flux de pyrolyse gazeux issu de l'étape d) et produisant au moins en un flux riche en composés légers, un flux riche en oligomères, et un flux riche en aromatiques.

Le flux riche en composés légers comprend majoritairement des composés plus légers que le benzène, en particulier les composés hydrogène, méthane, éthane, éthylène, propane, propylène, butane, butène, isobutane.

Le flux riche en aromatiques comprend majoritairement des composés aromatiques comprenant de 6 à 9 atomes de carbone. L'étape c) de séparation est opérée de telle manière que 99% en masse du styrène alimentant cette étape soit récupéré dans le flux riche en aromatiques.

Le flux riche en oligomères comprend majoritairement les oligomères du styrène. L'étape c) de séparation est opérée de manière à ce que le flux riche en oligomères comprenne moins de 5% en masse de composés aromatiques comprenant de 6 à 9 atomes de carbone. Outre qu'elle maximise la quantité de composés aromatiques récupérés dans le flux riche en aromatiques, la minimisation de la teneur en composés aromatiques comprenant de 6 à 9 atomes de carbone dans le flux riche en oligomères permet d'améliorer le fonctionnement de la section de pyrolyse dans l'étape d) de deuxième pyrolyse en minimisant la part d'énergie absorbée par l'évaporation de ces composés aromatiques dans la section de pyrolyse, évaporation qui peut entraîner des problèmes de moussage et donc impacter le bon fonctionnement de cette section.

De manière préférée, l'étape c) de séparation est mise en œuvre dans une colonne à distiller.

Dans cet arrangement, en tête de colonne, l'effluent vapeur est refroidi à une température comprise entre 30°C et 50°C, de préférence comprise entre 35°C et 45°C. La fraction liquide condensée est renvoyée en tête de colonne en tant que reflux, tandis que la fraction vapeur est ensuite sous-refroidie à une température comprise entre -5°C et 10°C, préférentiellement entre -5°C et 5°C afin de condenser le styrène éventuellement entrainé avec les composés légers. Le flux condensé après sous-refroidissement est renvoyé en tête de colonne en tant que reflux. La fraction vapeur résiduelle constitue le flux riche en composés légers. Ce flux peut ensuite être valorisé, par exemple sous forme énergétique. Un premier refroidissement permet d'utiliser au maximum de l'eau de refroidissement à température ambiante en tant qu'utilité froide et minimise l'utilisation d'utilité froide spécifique pour obtenir un sous-refroidissement, ce qui impacte favorablement l'analyse de cycle de vie du procédé selon l'invention.

Dans cet arrangement, la colonne à distiller est opérée à une pression comprise entre 0,1 et 2,0 bara, de préférence entre 0,5 et 1,5 bara et de manière préférée entre 0,5 et 1,1 bar, la pression d'opération étant entendue comme la pression mesurée en tête de colonne. Par « bara », on entend bar absolu, par opposition à une pression exprimée en bar relatif, communément noté « barg » selon la notation anglaise « bar gauge ».

Dans cet arrangement et de manière préférée, la colonne à distiller est alimentée en fond de colonne par au moins le premier flux de pyrolyse gazeux issu de l'étape b) et le deuxième flux de pyrolyse gazeux issu de l'étape d) et produit en tête de colonne un flux riche en composés légers, en fond un flux riche en oligomères, et par un soutirage latéral un flux riche en aromatiques, ladite colonne ayant pour seul apport de chaleur ledit premier flux de pyrolyse gazeux issu de l'étape b) et ledit deuxième flux de pyrolyse gazeux issu de l'étape d).

Le premier flux de pyrolyse gazeux issu de l'étape b) et le deuxième flux de pyrolyse gazeux issu de l'étape d) sont à température élevée, de préférence à une température supérieure à 300°C. Cette température est suffisante pour que la colonne ne nécessite pas d'autre apport de chaleur.

En alimentant la charge en fond de colonne, la charge est rapidement refroidie, permettant ainsi de limiter les réactions éventuelles de polymérisation du styrène. Cette alimentation permet en outre une meilleure gestion des composés dits lourds. En effet, l'absence de système de recirculation en fond de colonne, utilisé habituellement pour maintenir en température la colonne à distiller, limite grandement les risques d'encrassement par les composés dits lourds, particulièrement visqueux.

La colonne à distiller mise en œuvre dans l'étape c) du procédé selon l'invention comprend de 5 à 20 étages théoriques, de préférence au plus 15 étages théoriques, de manière préférée de 8 à 12 étages théoriques.

On soutire sur un plateau intermédiaire un flux riche en aromatiques. Ce plateau de soutirage est situé dans le tiers inférieur de la colonne à distiller, de préférence de 1 à 3 étages théoriques du plateau de fond. Ce soutirage à une position basse de la colonne, légèrement éloigné du plateau de fond, permet de limiter l'entrainement de composés lourds dans le flux riche en aromatiques et limite d'autant les risques d'encrassement des équipements ultérieurs.

De manière préférée, et afin de limiter encore plus le risque de polymérisation, un inhibiteur de polymérisation du styrène en polystyrène, tel que le 2,2,6,6-tetramethyl-4-oxopiperidinooxy, peut être alimenté dans la colonne à distiller de l'étape c) du procédé, de préférence au niveau de la tête de colonne.

### Étape d) de deuxième pyrolyse

Le procédé selon l'invention comprend une étape de deuxième pyrolyse comprenant une section de pyrolyse alimentée au moins par le flux riche en oligomères issu de l'étape c), et produisant au moins un deuxième flux de pyrolyse gazeux et un deuxième flux de pyrolyse liquide.

Dans cette étape, les oligomères du styrène sont convertis en composés mono-aromatiques, en particulier en monomère styrène. La section de pyrolyse est opérée à une température allant de 400°C à 900°C, de préférence de 500°C à 800°C, de manière préférée entre 650°C et 800°C. Lorsque la température est inférieure à 400°C, le rendement liquide et le rendement en composés mono-aromatiques sont insuffisants, tandis qu'au-delà de 900°C, ces rendements se dégradent. L'intervalle de température compris entre 650°C et 800°C permet, dans les conditions de l'invention, de maximiser à la fois le rendement liquide et le rendement en composés mono-aromatiques. Par rendement liquide, on entend le pourcentage massique de liquide obtenu après pyrolyse d'une masse donnée de flux riche en oligomères. Par rendement en composés mono-aromatiques, on entend le pourcentage massique de composés mono-aromatiques tels que le styrène, l'éthylbenzène, le toluène, le cumène, l'alpha-méthyl-styrène dans la fraction liquide obtenue après pyrolyse d'une masse donnée de flux riche en oligomères.

La section de pyrolyse est opérée à une pression allant de 1,0 bar à 7,5 bar, de préférence allant de 3 bar à 6 bar et de manière préférée allant de 3 bar à 4,5 bar. L'augmentation de la pression opératoire permet d'améliorer le rendement en composés mono-aromatiques en augmentant la tension de vapeur des différents constituants, mais dégrade le rendement liquide. Cet intervalle de pression permet, dans les conditions de l'invention, de maximiser à la fois le rendement liquide et le rendement en composés mono-aromatiques.

La section de pyrolyse est préférentiellement opérée avec un temps de séjour de la phase gaz allant de 10 s à 30 s, de manière préférée allant de 10 s à 25 s et de manière très préférée allant de 15 s à 20 s, le temps de séjour étant défini comme le rapport du volume (en m³) du réacteur (ou des réacteurs, s'il y en a plusieurs en série) de la section de pyrolyse et des conduites acheminant le deuxième flux de pyrolyse gazeux jusqu'à l'étape c) de séparation sur le débit volumique (en m³/s) du deuxième flux de pyrolyse gazeux. Cet intervalle préféré de temps de séjour permet de maximiser le rendement en composés mono-aromatiques. En dessous de 10 s, le temps de séjour est trop faible pour permettre une conversion suffisante, tandis qu'au-delà de 30 s, une possible recombinaison des composés mono-aromatiques dégrade le rendement. Le rendement en composés mono-aromatiques est particulièrement maximisé pour un temps de séjour allant de 15 s à 20 s. Le temps de séjour de la phase gaz peut être ajusté par exemple en alimentant un gaz inerte dans la section de pyrolyse de l'étape d), par exemple un gaz choisi parmi l'azote, l'argon, l'hélium, le néon, le xénon, le krypton, préférentiellement l'azote.

La demanderesse a découvert que le rendement en composés mono-aromatiques, en particulier en monomère styrène, pouvait être contrôlé en ajustant la pression partielle en hydrogène de la section de pyrolyse de l'étape d) et/ou en contrôlant le temps de séjour de la phase gaz. Cette pression partielle est contrôlée en ajustant la teneur en agent donneur d'hydrogène dans la charge polystyrénique et/ou dans l'alimentation de la section de pyrolyse de l'étape d).

La pression partielle en hydrogène de la section de pyrolyse de l'étape d) peut être augmentée en augmentant la teneur en agent donneur d'hydrogène dans la charge polystyrénique et/ou dans l'alimentation de la section de pyrolyse de l'étape d), ou diminuée par exemple en alimentant un gaz inerte dans la section de pyrolyse de l'étape d), par exemple un gaz choisi parmi l'azote, l'argon, l'hélium, le néon, le xénon, le krypton, préférentiellement l'azote. La teneur en agent donneur d'hydrogène peut être augmentée en mélangeant à la charge polystyrénique et/ou à l'alimentation de la section de pyrolyse de l'étape d) un ou plusieurs agents donneurs d'hydrogène, la charge polystyrénique et/ou l'alimentation de la section de pyrolyse de l'étape d) pouvant déjà contenir un ou plusieurs agents donneurs d'hydrogène.

Ainsi, dans un arrangement, la pression partielle en hydrogène dans la section de pyrolyse de l'étape d) peut être ajustée par la présence d'un agent donneur d'hydrogène choisi parmi les polyoléfines, les cires paraffiniques et leurs mélanges dans la charge polystyrénique et/ou dans le flux riche en oligomères issu de l'étape c).

Dans un autre arrangement, associé ou non à l'arrangement précédent, la pression partielle en hydrogène peut également être ajustée par la présence d'un agent donneur d'hydrogène choisi parmi les polyoléfines, les cires paraffiniques, le dihydrogène et leurs mélanges dans l'alimentation de la section de pyrolyse de l'étape d).

En ajustant la teneur en agent donneur d'hydrogène dans le procédé selon l'invention et/ou le temps de séjour de la phase gaz, il est ainsi possible de contrôler le rendement global en composés mono-aromatiques. La teneur en agent donneur d'hydrogène peut être ajustée en mesurant la teneur en composé mono-aromatique dans le deuxième flux de pyrolyse gazeux, la teneur optimale pouvant être légèrement différente suivant que l'on souhaite, par exemple, maximiser la production de toluène et d'éthylbenzène, ou de styrène.

Par exemple, dans le cas où le donneur d'hydrogène est le polypropylène, le ratio massique polypropylène sur polystyrène dans la charge polystyrénique sera ajusté entre 1% poids et 15% poids, de préférence entre 1% et 3% poids.

En présence d'une teneur trop importante en agent donneur d'hydrogène, le rendement en produits légers non aromatiques (hydrogène, méthane, éthane, éthylène, propane, propylène, butane, butène, isobutane) et en résidus solides issus de la dégradation thermique des composés lors de la pyrolyse augmente, ce qui grève le rendement global en produits d'intérêt.

De préférence, la section de pyrolyse met en œuvre une section de pyrolyse par micro-onde. Une telle pyrolyse par micro-onde utilisable pour la pyrolyse d'une charge polystyrénique est par exemple décrite dans le document WO 2020/202089.

### Étape e) de séparation

Le procédé selon l'invention comprend une étape de séparation du flux riche en aromatiques en au moins un flux comprenant majoritairement de l'éthylbenzène, un flux comprenant majoritairement du styrène et un flux de composés lourds.

L'étape e) de séparation permet d'obtenir un flux comprenant majoritairement du styrène pouvant alimenter un procédé de polymérisation du styrène, donc répondant aux spécifications de tels procédés, avec en particulier une teneur en styrène très élevée, de préférence supérieure à 99,8% poids, et des teneurs en composés tels que éthylbenzène, benzène, cumène, alpha-méthyl-styrène et oligomères du styrène très faibles.

Dans un premier arrangement préféré, l'étape e) de séparation du flux riche en aromatiques comprend deux sections de séparations successives.

Une première section de séparation est alimentée par le flux riche en aromatiques issu de l'étape c) et permet de séparer un flux comprenant majoritairement de l'éthylbenzène et un raffinat styrénique.

Cette première section est mise en œuvre dans une colonne de distillation comprenant de 60 à 100 étages théoriques, et est opérée à une pression inférieure ou égale à 0,25 bara en tête de colonne de manière à maintenir la température en fond de colonne à une valeur inférieure ou égale à 120°C.

La colonne de distillation de la première section est alimentée par le flux riche en aromatiques issu de l'étape c) en bas du tiers supérieur de la colonne. Par exemple, pour une colonne comprenant 60 étages théoriques, le flux riche en aromatiques est alimenté à un étage compris entre le 18^{ème} étage théorique et le 22^{ème} étage théorique, les étages étant numérotés de haut en bas.

Le taux de reflux au condensateur de cette colonne, correspondant au débit massique de reflux alimenté en tête de colonne sur le débit massique de flux comprenant majoritairement de l'éthylbenzène est préférentiellement compris entre 60 et 300. Ce paramètre varie fortement en fonction de la teneur en éthylbenzène du flux riche en aromatiques. Plus la teneur en éthylbenzène dans le flux riche en aromatiques sera faible, plus le taux de reflux au condensateur de la colonne sera élevé.

Le taux de reflux au rebouilleur de cette colonne, correspondant au débit massique de reflux alimenté en fond de colonne sur le débit massique de raffinat styrénique est préférentiellement compris entre 4 et 10, de manière préférée compris entre 5 et 9.

Une deuxième section de séparation est alimentée par le raffinat styrénique issu de la première section de séparation et produit un flux comprenant majoritairement du styrène et un flux de composés lourds.

Cette deuxième section est mise en œuvre dans une colonne de distillation comprenant de 40 à 100 étages théoriques, de manière préférée comprenant de 40 à 70 étages théoriques, et est opérée à une pression inférieure ou égale à 0,25 bara en tête de colonne de manière à maintenir la température en fond de colonne à une valeur inférieure ou égale à 120°C.

La colonne de distillation de la première section est alimentée par le raffinat styrénique issu de la première section de séparation dans la partie inférieure de la colonne, préférentiellement dans le haut du cinquième inférieur de la colonne. Par exemple, pour une colonne comprenant 50 étages théoriques, le raffinat styrénique issu de la première section de séparation est alimenté à un étage compris entre le 35^{ème} étage théorique et le 45^{ème} étage théorique, les étages étant numérotés de haut en bas.

Le taux de reflux au condensateur de cette colonne, correspondant au débit massique de reflux alimenté en tête de colonne sur le débit massique de flux comprenant majoritairement du styrène est préférentiellement compris entre 4 et 8.

Le taux de reflux au rebouilleur de cette colonne, correspondant au débit massique de reflux alimenté en fond de colonne sur le débit massique de flux de composés lourds est préférentiellement compris entre 40 et 200, ce ratio étant grandement influencé par la teneur en composés tels que cumène et alpha-méthylstyrène.

Dans un autre arrangement préféré, l'étape e) de séparation du flux riche en aromatiques est mise en œuvre dans une colonne à distiller à paroi interne.

Une colonne à paroi interne est un équipement de distillation bien connu de l'homme du métier dans lequel une paroi interne étanche aux fluides et disposée verticalement sépare une partie de la colonne en deux zones distinctes. Une colonne à paroi interne est ainsi généralement constituée d'une partie commune inférieure dans laquelle les étages de séparation ne sont pas divisés par la paroi interne, d'une partie divisée dans laquelle les étages de séparation sont divisés par la paroi interne et d'une partie commune supérieure dans laquelle les étages de séparation ne sont pas divisés par la paroi interne.

La colonne à paroi interne comprend au total de 70 à 130 étages théoriques, préférentiellement de 80 à 120 étages théoriques, très préférentiellement de 90 à 110 étages théoriques. La paroi interne est préférentiellement centrée, c'est-à-dire qu'elle partitionne la colonne dans la longueur où elle est présente en deux parties de volume égal. Lorsque le nombre d'étages théoriques de part et d'autre de la paroi interne est différent, par exemple du fait de l'utilisation de types de plateaux distributeurs ou de garnissage différent, le nombre total d'étages théoriques de la colonne est entendu comme la somme des étages théoriques des parties communes et du plus grand nombre d'étages entre les deux parties divisées. La colonne est opérée à une pression inférieure ou égale à 0,25 bara en tête de colonne de manière à maintenir la température en fond de colonne à une valeur inférieure ou égale à 120°C.

Le flux riche en aromatiques issu de l'étape c) du procédé est alimenté d'un côté de la paroi interne sur un étage allant du 10^{ème} au 20^{ème} étage théorique, de préférence allant du 12^{ème} au 18^{ème} étage théorique et très préférentiellement au 15^{ème} étage théorique, les étages étant numérotés de haut en bas.

Dans une première variante de cet arrangement, la partie inférieure commune de la colonne à paroi interne comprend de 8 à 12 étages théoriques et la partie commune supérieure comprend de 8 à 12 étages théoriques. Le flux comprenant majoritairement du styrène est soutiré dans la partie opposée, par rapport à la paroi interne, à la partie où est injecté le flux riche en aromatiques. Le soutirage est effectué sur un étage proche de la partie supérieure de la partie divisée, préférentiellement sur l'un des 5 étages supérieurs de la partie divisée, de manière préférée sur l'un des 3 étages supérieurs de la partie divisée de manière très préférée sur l'un des deux étages supérieurs de la partie divisée, et très préférentiellement sur le premier étage de la partie divisée, en comptant les étages à partir du haut.

On soutire en tête de colonne le flux comprenant majoritairement de l'éthylbenzène et en fond de colonne le flux de composés lourds.

Le taux de reflux au condensateur de cette colonne, correspondant au débit massique de reflux alimenté en tête de colonne sur le débit massique de flux comprenant majoritairement de l'éthylbenzène est préférentiellement compris entre 60 et 300. Ce paramètre varie fortement en fonction de la teneur en éthylbenzène du flux riche en aromatiques. Plus la teneur en éthylbenzène dans le flux riche en aromatiques sera faible, plus le taux de reflux au condensateur de la colonne sera élevé.

Le taux de reflux au rebouilleur de cette colonne, correspondant au débit massique de reflux alimenté en fond de colonne sur le débit massique de flux de composés lourds est préférentiellement compris entre 50 et 200, ce ratio étant grandement influencé par la teneur en composés tels que cumène et alpha-méthylstyrène.

Dans une deuxième variante de cet arrangement, la colonne à paroi interne ne comprend pas de partie commune supérieure. C'est-à-dire que la paroi s'étend jusqu'à la tête de la colonne à paroi interne.

Dans cette variante, la partie commune inférieure comprend de 2 à 12 étages théoriques, préférentiellement de 2 à 10 étages théoriques, et très préférentiellement de 2 à 4 étages théoriques.

Dans cette variante, la colonne à paroi interne comprend préférentiellement au total de 60 à 80 étages théoriques.

Dans cette variante, le flux comprenant majoritairement du styrène est soutiré dans la partie opposée, par rapport à la paroi interne, à la partie où est injecté le flux riche en aromatiques. Le soutirage est effectué en tête de colonne.

Le flux comprenant majoritairement de l'éthylbenzène est soutiré en tête de colonne dans la même partie que la partie où est injecté le flux riche en aromatiques.

Le taux de reflux au condensateur de cette colonne pour la partie divisée située du côté de l'alimentation en flux riche en aromatiques, correspondant au débit massique de reflux alimenté en tête de colonne dans cette partie sur le débit massique de flux comprenant majoritairement de l'éthylbenzène est préférentiellement compris entre 60 et 300. Ce paramètre varie fortement en fonction de la teneur en éthylbenzène du flux riche en aromatiques. Plus la teneur en éthylbenzène dans le flux riche en aromatiques sera faible, plus le taux de reflux au condensateur de la colonne sera élevé.

Le taux de reflux au condensateur de cette colonne pour la partie divisée située du côté du soutirage du flux comprenant majoritairement du styrène, correspondant au débit massique de reflux alimenté en tête de colonne sur le débit massique de flux comprenant majoritairement du styrène est préférentiellement compris entre 1 et 10.

Le taux de reflux au rebouilleur de cette colonne, correspondant au débit massique de reflux alimenté en fond de colonne sur le débit massique de flux de composés lourds est préférentiellement compris entre 60 et 200, ce ratio étant grandement influencé par la teneur en composés tels que cumène et alpha-méthylstyrène.

De manière préférée, et afin de limiter encore plus le risque de polymérisation, un inhibiteur de polymérisation du styrène en polystyrène, tel que le 2,2,6,6-tetramethyl-4-oxopiperidinooxy, peut être alimenté dans la, ou les colonnes mises en œuvre dans l'étape b) de séparation, préférentiellement en tête de la, ou des colonnes mises en œuvre dans l'étape b) de séparation.

De manière préférée, le flux comprenant majoritairement de l'éthylbenzène issu de l'étape e) est séparé en au moins un flux comprenant majoritairement du toluène, un flux qui comprend de l'éthylbenzène et un flux qui comprend du styrène.

De manière préférée le flux de composés lourds issu de l'étape e) est séparé en au moins un flux comprenant du styrène, un flux qui comprend de l'AMS et un flux qui comprend des oligomères.

### Description des figures

Pour chacune des figures suivantes, les numéros et lettres identiques correspondent à des flux et des opérations similaires.

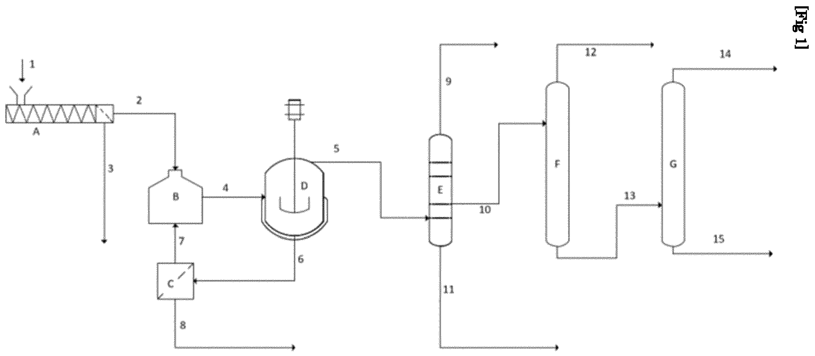

[Fig 1] La figure 1 illustre schématiquement un procédé de dépolymérisation d'une charge polystyrénique selon l'art antérieur.

Une charge polystyrénique (1) alimente une section d'extrusion dans laquelle elle est progressivement mise en température de manière à fondre ladite charge (A), la partie liquide (2) étant séparée de la partie solide (3). La partie liquide (2) est envoyée dans un bac de mélange (B) où elle est mélangée à la fraction liquide (7) issue du séparateur liquide-solide (C).

Le bac de mélange (B) alimente le réacteur de pyrolyse (D), lequel produit un premier flux de pyrolyse gazeux (5) et un premier flux de pyrolyse liquide (6), ce dernier étant séparé en une fraction solide (8) éventuellement présente et en une fraction liquide (7) dans le séparateur liquide-solide (C).

Le premier flux de pyrolyse gazeux (5) alimente une première colonne à distiller (E) qui produit en tête un flux riche en composés légers (9), en fond un flux riche en oligomères (11) et en tant que soutirage latéral un flux riche en aromatiques (10).

Ce dernier alimente une colonne à distiller (F) dans laquelle il est séparé en un flux comprenant majoritairement de l'éthylbenzène (12) et un raffinat styrénique (13) qui alimente une colonne à distiller (G) le séparant en un flux comprenant majoritairement du styrène (14) et un flux de composés lourds (15).

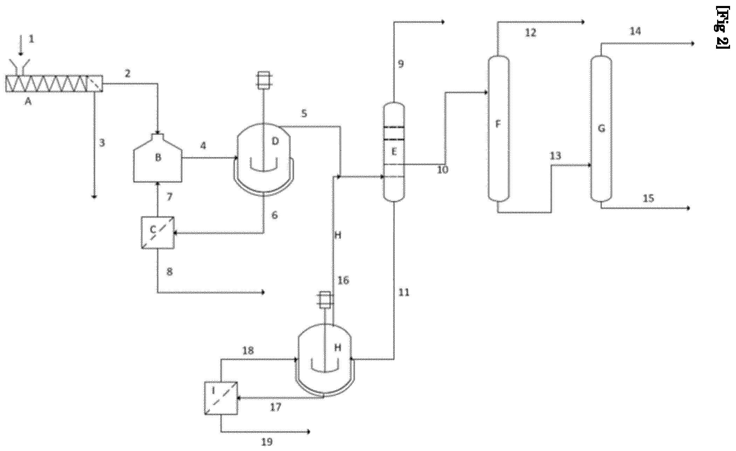

[Fig 2] La figure 2 illustre schématiquement une configuration possible d'un procédé de dépolymérisation d'une charge polystyrénique selon l'invention.

Les éléments fonctionnellement identiques à ceux de la figure 1 sont numérotés de la même manière, sans que cela ne signifie qu'ils aient le même dimensionnement ou soient opérés dans les mêmes conditions.

Dans cet arrangement, le flux riche en oligomères (11) alimente un réacteur de pyrolyse (H) qui produit un deuxième flux de pyrolyse gazeux (16), ce dernier étant mélangé avec le premier flux de pyrolyse gazeux (5) avant d'alimenter la colonne à distiller (E). Le réacteur de pyrolyse (H) produit également un deuxième flux de pyrolyse liquide (17). Le deuxième flux de pyrolyse liquide (17) est séparé en une fraction solide (19) éventuellement présente dans le séparateur liquide-solide (I) et une fraction liquide (18) qui est alimentée dans le réacteur de pyrolyse (H).

Dans cet arrangement, et de manière non représentée sur ce schéma, on peut ajuster le fonctionnement du réacteur de pyrolyse (H) par l'injection d'un gaz inerte en mélange avec le flux riche en oligomères (11) ou contrôler la pression partielle en hydrogène du réacteur de pyrolyse (H) par la présence d'un agent donneur d'hydrogène dans la charge polystyrénique (1) et/ou dans le flux riche en oligomères (11).

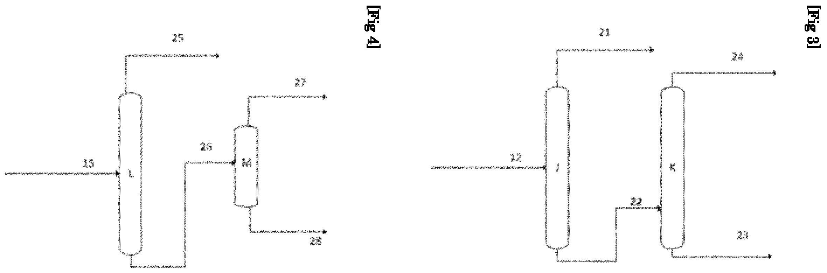

[Fig.3] La figure 3 illustre schématiquement un arrangement dans lequel un flux comprenant majoritairement de l'éthylbenzène (12) alimente une colonne à distiller (J) comprenant préférentiellement de 20 à 40 étages théoriques, ici 30, substantiellement en son milieu, ici au plateau théorique 30. Cette colonne (J) produit un flux comprenant majoritairement du toluène (21) et un raffinat (22). Ce dernier alimente une colonne à distiller (K) comprenant préférentiellement de 50 à 80 étages théoriques, ici 65, dans la partie supérieure de sa moitié inférieure, ici au plateau théorique 30. Cette colonne (K) produit un flux (24) qui comprend de l'éthylbenzène et un flux (23) qui comprend du styrène. Cet arrangement permet de maximiser la récupération de ces constituants.

[Fig.4] La figure 4 illustre schématiquement un arrangement dans lequel un flux de composés lourds (15) est traité afin de maximiser la récupération en styrène et en alpha-méthyl-styrène (AMS). Le flux de composés lourds (15) alimente une colonne à distiller (L) comprenant préférentiellement de 15 à 25 étages théoriques, ici 20, substantiellement en son milieu, ici au plateau théorique 10. Cette colonne (L) produit un flux (25) comprenant du styrène et un raffinat (26). Ce dernier alimente une colonne à distiller (M) comprenant de 1 à 3 étages théoriques, ici un flash comprenant un seul étage théorique, substantiellement en son milieu lorsque la colonne comprend plusieurs étages théoriques. Cette colonne (M) produit un flux (27) qui comprend de l'AMS et un flux (28) qui comprend des oligomères.

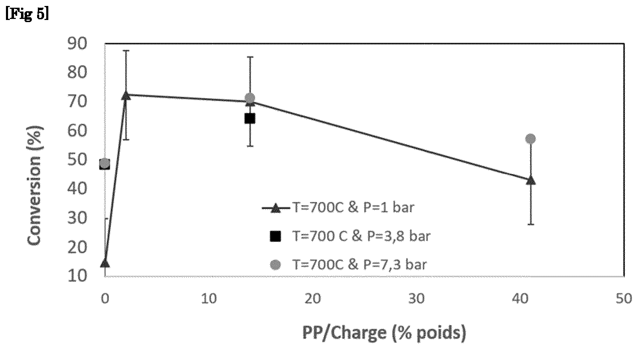

[Fig.5] La figure 5 est une représentation de la conversion des oligomères en fonction de la fraction massique de donneur d'hydrogène, ici du polypropylène ajouté dans la charge polystyrénique, calculé comme le ratio du débit de polypropylène sur la somme du débit de polypropylène et du débit de polystyrène dans la charge polystyrénique, à une température de 700°C pour différentes pressions opératoires dans l'étape de pyrolyse.

### Exemples

### Exemple 1

Cet exemple illustre la dépolymérisation du polystyrène selon le schéma de la figure 1.

Une charge polystyrénique (1), ici du polystyrène, alimente une extrudeuse (A) dans laquelle elle est mise à une température de 250°C. La partie liquide de la charge (2) alimente, après mélange avec la partie liquide issue du réacteur de pyrolyse (7), un réacteur de pyrolyse (D), ici une pyrolyse par micro-onde, opérée à une température de 340°C et à une pression de 1,1 bar. Le premier flux de pyrolyse gazeux (5) est séparé en un flux riche en composés légers (9), un flux riche en oligomères (11), un flux comprenant majoritairement de l'éthylbenzène (12), un flux comprenant majoritairement du styrène (14) et un flux de composés lourds (15).

Les caractéristiques des colonnes à distiller E, F et G sont présentées dans le tableau 1.

Les productions en styrène, toluène, alpha-méthyl-styrène et autres aromatiques sont indiquées dans le tableau 2.

### Exemple 2

Cet exemple illustre la dépolymérisation du polystyrène selon l'invention, selon le schéma représenté figure 2, les flux (12) et (15) étant traités selon les schémas présentés dans les figures 3 et 4.

Le réacteur de pyrolyse (D), ici une pyrolyse micro-onde, est opéré à une température de 340°C et une pression de 1,1 bar. Le deuxième réacteur de pyrolyse (H), ici une pyrolyse micro-onde, est opéré à une température de 700°C et une pression de 3,7 bar.

Les caractéristiques des colonnes à distiller E, F, G, J, K, L et M sont présentées dans le tableau 1.

On montre dans le tableau 2 les résultats en terme de production globale dans trois cas :
- Exemple 2a : la charge polystyrénique comprend 2% poids de polypropylène
- Exemple 2b : la charge polystyrénique comprend 14% poids de polypropylène

Le polypropylène agit comme donneur d'hydrogène. On observe que la présence d'un donneur d'hydrogène dans la charge permet d'améliorer le rendement global en composés aromatiques d'intérêt, et notamment en styrène. Toutefois, une présence en des proportions trop importantes nuira au rendement global.

**[Tableau 1]**

| **Colonne** | **Unité** | **E** | **F** | **G** | **J** | **K** | **L** | **M** |
|---|---|---|---|---|---|---|---|---|
| **Nombre d'étages théoriques** | - | 10 | 60 | 50 | 30 | 65 | 20 | 1 |
| **Etage alimentation** | - | 8 | 20 | 25 | 15 | 30 | 10 | 1 |
| **Pression condensateur** | **mbar** | 1013 | 77 | 47 | 200 | 200 | 85 | 85 |
| **Température condensateur** | **°C** | 2 | 35 | 35 | 61 | 85 | 72 | 88 |
| **Température rebouilleur** | **°C** | 224 | 80 | 80 | 87 | 92 | 88 | 95 |

**[Tableau 2]**

| **Pour 100 kg/h de PS pur** | **Exemple 1** | **Exemple 2a** | **Exemple 2b** |
|---|---|---|---|
| | | **PP/Charge 2% poids** | **PP/Charge 14% poids** |
| **Prod Styrene** | 58.6 | 60.0 | 60.6 |
| **Prod Toluene** | 1.8 | 5.3 | 5.1 |
| **Prod AMS** | 9.2 | 9.8 | 10.1 |
| **Prod autres aromatiques** | 2.6 | 5.3 | 5.2 |
| **Total Aromatiques** | **72.1** | **80.3** | **81.0** |
| **Prod Incondensable** | 8.4 | 13.6 | 12.9 |
| **Prod Oligomères légers** | 16.8 | 0.0 | 0.0 |
| **Prod résidus solides** | 2.7 | 6.1 | 6.1 |

## Revendications

1. Procédé de dépolymérisation d'une charge polystyrénique comprenant au moins les étapes suivantes :
a. Une étape de préparation de la charge polystyrénique afin de provoquer la fonte des composés plastiques ;
b. Une étape de première pyrolyse de la charge issue de l'étape a) comprenant une section de pyrolyse et produisant au moins un premier flux de pyrolyse gazeux et un premier flux de pyrolyse liquide ;
c. Une étape de séparation alimentée au moins par le premier flux de pyrolyse gazeux issu de l'étape b) et le deuxième flux de pyrolyse gazeux issu de l'étape d) et produisant au moins un flux riche en composés légers, un flux riche en oligomères, et un flux riche en aromatiques ;
d. Une étape de deuxième pyrolyse comprenant une section de pyrolyse alimentée au moins par le flux riche en oligomères issu de l'étape c), et produisant au moins un deuxième flux de pyrolyse gazeux et un deuxième flux de pyrolyse liquide ;
e. Une étape de séparation du flux riche en aromatiques issu de l'étape c) en au moins un flux comprenant majoritairement de l'éthylbenzène, un flux comprenant majoritairement du styrène et un flux de composés lourds ;
dans lequel on contrôle, dans la section de pyrolyse de l'étape d), la pression partielle en hydrogène et le temps de séjour de la phase gaz, et dans lequel la pression partielle en hydrogène de la section de pyrolyse de l'étape d) est contrôlée par la présence d'un agent donneur d'hydrogène dans la charge polystyrénique et/ou dans l'alimentation de la section de pyrolyse de l'étape d) et/ou par l'alimentation d'un gaz inerte dans la section de pyrolyse de l'étape d).

2. Procédé de dépolymérisation selon la revendication précédente dans lequel la pression partielle en hydrogène de la section de pyrolyse de l'étape d) est contrôlée par la présence d'un agent donneur d'hydrogène choisi parmi les polyoléfines, les cires paraffiniques et leurs mélanges dans la charge polystyrénique et/ou dans le flux riche en oligomères issu de l'étape c).

3. Procédé de dépolymérisation selon la revendication précédente dans lequel la pression partielle en hydrogène de la section de pyrolyse de l'étape d) est contrôlée par la présence d'un donneur d'hydrogène dans la charge styrénique, le donneur d'hydrogène étant le polypropylène, le ratio massique polypropylène sur polystyrène dans la charge polystyrénique étant ajusté entre 1% poids et 15% poids, de préférence entre 1% et 3% poids.

4. Procédé de dépolymérisation selon l'une quelconque des revendications 1 à 3 dans lequel la pression partielle en hydrogène de la section de pyrolyse de l'étape d) est contrôlée par la présence d'un agent donneur d'hydrogène choisi parmi les polyoléfines, les cires paraffiniques, le dihydrogène et leurs mélanges dans l'alimentation de la section de pyrolyse de l'étape d).

5. Procédé de dépolymérisation selon l'une quelconque des revendications précédentes dans lequel l'étape d) de deuxième pyrolyse est opérée à une température allant de 400°C à 900°C, de préférence de 500°C à 800°C, de manière préférée entre 650°C et 800°C, et à une pression allant de 1,0 bar à 7,5 bar, de préférence allant de 3 bar à 6 bar et de manière préférée allant de 3 bar à 4,5 bar.

6. Procédé de dépolymérisation selon l'une quelconque des revendications précédentes dans lequel l'étape d) de deuxième pyrolyse est opérée avec un temps de séjour de la phase gaz allant de 10 s à 30 s, de manière préférée allant de 10 s à 25 s et de manière très préférée allant de 15 s à 20 s.

7. Procédé de dépolymérisation selon la revendication précédente dans lequel le temps de séjour de la phase gaz est contrôlé en alimentant un gaz inerte dans la section de pyrolyse de l'étape d), de préférence un gaz inerte choisi parmi l'azote, l'argon, l'hélium, le néon, le xénon, le krypton, préférentiellement l'azote.

8. Procédé de dépolymérisation selon l'une quelconque des revendications précédentes dans lequel l'étape b) de première pyrolyse et/ou l'étape d) de deuxième pyrolyse met en œuvre une section de pyrolyse par micro-onde.

9. Procédé de dépolymérisation selon l'une quelconque des revendications précédentes dans lequel l'étape c) de séparation met en œuvre une colonne de distillation alimentée en fond de colonne par le premier flux de pyrolyse gazeux issu de l'étape b) et le deuxième flux de pyrolyse gazeux issu de l'étape d) et produisant en tête de colonne un flux riche en composés léger, en fond un flux riche en oligomères, et par un soutirage latéral un flux riche en aromatiques, ladite colonne ayant pour seul apport de chaleur ledit premier flux de pyrolyse gazeux issu de l'étape b) et ledit deuxième flux de pyrolyse gazeux issu de l'étape d).

10. Procédé de dépolymérisation selon l'une quelconque des revendications précédentes dans lequel l'étape e) de séparation du flux riche en aromatiques comprend une première section de séparation alimentée par le flux riche en aromatiques issu de l'étape c) qui permet de séparer un flux comprenant majoritairement de l'éthylbenzène et un raffinat styrénique et une deuxième section de séparation alimentée par le raffinat styrénique issu de la première section de séparation et produisant un flux comprenant majoritairement du styrène et un flux de composés lourds.

11. Procédé de dépolymérisation selon l'une quelconque des revendications précédentes dans lequel l'étape e) de séparation du flux riche en aromatiques est mise en œuvre dans une colonne à distiller à paroi interne.

12. Procédé de dépolymérisation selon l'une quelconque des revendications précédentes dans lequel le flux comprenant majoritairement de l'éthylbenzène issu de l'étape e) est séparé en au moins un flux comprenant majoritairement du toluène, un flux qui comprend de l'éthylbenzène et un flux qui comprend du styrène.

13. Procédé de dépolymérisation selon l'une quelconque des revendications précédentes dans lequel le flux de composés lourds issu de l'étape e) est séparé en au moins un flux comprenant du styrène, un flux qui comprend de l'AMS et un flux qui comprend des oligomères.

## Patentansprüche

1. Verfahren zur Depolymerisation eines Polystyrol-Einsatzmaterials, das zumindest die folgenden Schritte umfasst:
a. einen Schritt der Herstellung des Polystyrol-Einsatzmaterials, um das Schmelzen der Kunststoffverbindungen zu bewirken;
b. einen Schritt einer ersten Pyrolyse des Einsatzmaterials aus Schritt a), der einen Pyrolyseabschnitt umfasst und bei dem mindestens ein erster gasförmiger Pyrolysestrom und ein erster flüssiger Pyrolysestrom erzeugt werden;
c. einen Trennschritt, der zumindest von dem ersten gasförmigen Pyrolysestrom aus Schritt b) und dem zweiten gasförmigen Pyrolysestrom aus Schritt d) gespeist wird und bei dem mindestens ein an leichten Verbindungen reicher Strom, ein an Oligomeren reicher Strom und ein an Aromaten reicher Strom erzeugt werden;
d. einen Schritt einer zweiten Pyrolyse, der einen Pyrolyseabschnitt umfasst, der zumindest von dem an Oligomeren reichen Strom aus Schritt c) gespeist wird und bei dem mindestens ein zweiter gasförmiger Pyrolysestrom und ein zweiter flüssiger Pyrolysestrom erzeugt werden;
e. einen Schritt zur Trennung des an Aromaten reichen Stroms aus Schritt c) in mindestens einen überwiegend Ethylbenzol umfassenden Strom, einen überwiegend Styrol umfassenden Strom und einen Strom von schweren Verbindungen;
wobei im Pyrolyseabschnitt von Schritt d) der Wasserstoffpartialdruck und die Verweilzeit der Gasphase gesteuert werden und wobei der Wasserstoffpartialdruck des Pyrolyseabschnitts von Schritt d) durch das Vorhandensein eines wasserstoffabgebenden Mittels im Polystyrol-Einsatzmaterial und/oder in der Zufuhr des Pyrolyseabschnitts von Schritt d) und/oder durch die Zufuhr eines Inertgases im Pyrolyseabschnitt von Schritt d) gesteuert wird.

2. Depolymerisationsverfahren nach dem vorhergehenden Anspruch, wobei der Wasserstoffpartialdruck des Pyrolyseabschnitts von Schritt d) durch das Vorhandensein eines aus Polyolefinen, Paraffinwachsen und Mischungen davon ausgewählten wasserstoffabgebenden Mittels im Polystyrol-Einsatzmaterial und/oder im an Oligomeren reichen Strom aus Schritt c) gesteuert wird.

3. Depolymerisationsverfahren nach dem vorhergehenden Anspruch, wobei der Wasserstoffpartialdruck des Pyrolyseabschnitts von Schritt d) durch das Vorhandensein eines Wasserstoffdonors im Styrol-Einsatzmaterial gesteuert wird, es sich bei dem Wasserstoffdonor um Polypropylen handelt, das Gewichtsverhältnis von Polypropylen zu Polystyrol im Polystyrol-Einsatzmaterial zwischen 1 Gew.-% und 15 Gew.-%, vorzugsweise zwischen 1 Gew.-% und 3 Gew.-%, eingestellt wird.

4. Depolymerisationsverfahren nach einem der Ansprüche 1 bis 3, wobei der Wasserstoffpartialdruck des Pyrolyseabschnitts von Schritt d) durch das Vorhandensein eines aus Polyolefinen, Paraffinwachsen, Wasserstoff und Mischungen davon ausgewählten wasserstoffabgebenden Mittels in der Zufuhr des Pyrolyseabschnitts von Schritt d) gesteuert wird.

5. Depolymerisationsverfahren nach einem der vorhergehenden Ansprüche, wobei Schritt d) der zweiten Pyrolyse bei einer Temperatur von 400 °C bis 900 °C, vorzugsweise von 500 °C bis 800 °C, bevorzugt zwischen 650 °C und 800 °C, und bei einem Druck von 1,0 bar bis 7,5 bar, vorzugsweise zwischen 3 bar und 6 bar und bevorzugt von 3 bar bis 4,5 bar, betrieben wird.

6. Depolymerisationsverfahren nach einem der vorhergehenden Ansprüche, wobei Schritt d) der zweiten Pyrolyse mit einer Verweilzeit der Gasphase von 10 s bis 30 s, bevorzugt von 10 s bis 25 s und außerordentlich bevorzugt zwischen 15 s und 20 s, betrieben wird.

7. Depolymerisationsverfahren nach dem vorhergehenden Anspruch, wobei die Verweilzeit der Gasphase gesteuert wird, indem ein Inertgas, vorzugsweise ein aus Stickstoff, Argon, Helium, Neon, Xenon, Krypton ausgewähltes Inertgas, vorzugsweise Stickstoff, in den Pyrolyseabschnitt von Schritt d) eingeführt wird.

8. Depolymerisationsverfahren nach einem der vorhergehenden Ansprüche, wobei in Schritt b) der ersten Pyrolyse und/oder Schritt d) der zweiten Pyrolyse ein Mikrowellen-Pyrolyseabschnitt verwendet wird.

9. Depolymerisationsverfahren nach einem der vorhergehenden Ansprüche, wobei im Trennschritt c) eine Destillationskolonne verwendet wird, die am Boden der Kolonne vom ersten gasförmigen Pyrolysestrom aus Schritt b) und dem zweiten gasförmigen Pyrolysestrom aus Schritt d) gespeist wird und am Kopf der Kolonne ein an leichten Verbindungen reicher Strom, am Boden ein an Oligomeren reicher Strom und durch eine seitliche Abnahme ein an Aromaten reicher Strom erzeugt werden, wobei die Kolonne als einzige Wärmezufuhr den ersten gasförmigen Pyrolysestrom aus Schritt b) und den zweiten gasförmigen Pyrolysestrom aus Schritt d) aufweist.

10. Depolymerisationsverfahren nach einem der vorhergehenden Ansprüche, wobei Schritt e) zur Trennung des an Aromaten reichen Stroms einen ersten Trennabschnitt, der von dem an Aromaten reichen Strom aus Schritt c) gespeist wird, der die Trennung eines überwiegend Ethylbenzol umfassenden Stroms und eines Styrolraffinats ermöglicht, und einen zweiten Trennschritt umfasst, der von dem Stryolraffinat aus dem ersten Trennabschnitt gespeist wird und bei dem ein Strom erzeugt wird, der überwiegend Styrol und einen Fluss von schweren Verbindungen umfasst.

11. Depolymerisationsverfahren nach einem der vorhergehenden Ansprüche, wobei Schritt e) zur Trennung des an Aromaten reichen Stroms in einer Destillationskolonne mit Innenwand erfolgt.

12. Depolymerisationsverfahren nach einem der vorhergehenden Ansprüche, wobei der überwiegend Ethylbenzol umfassende Strom aus Schritt e) in mindestens einen überwiegend Toluol umfassenden Strom, einen Ethylbenzol umfassenden Strom und einen Styrol umfassenden Strom getrennt wird.

13. Depolymerisationsverfahren nach einem der vorhergehenden Ansprüche, wobei der Fluss von schweren Verbindungen aus Schritt e) in mindestens einen Styrol umfassenden Strom, einen AMS umfassenden Strom und einen Oligomere umfassenden Strom getrennt wird.

## Claims

1. Process for depolymerizing a polystyrene feedstock, comprising at least the following steps:
a. a step of preparing the polystyrene feedstock so as to bring about the melting of the plastic compounds,
b. a step of performing a first pyrolysis of the feedstock resulting from step a) involving a pyrolysis section and producing at least a first gaseous pyrolysis stream and a first liquid pyrolysis stream,
c. a separating step supplied at least by the first gaseous pyrolysis stream from step b) and the second gaseous pyrolysis stream from step d) and producing at least a stream rich in light compounds, a stream rich in oligomers, and a stream rich in aromatics,
d. a step of performing a second pyrolysis involving a pyrolysis section supplied at least by the stream rich in oligomers from step c) and producing at least a second gaseous pyrolysis stream and a second liquid pyrolysis stream,
e. a step of separating the stream rich in aromatics from step c) into at least a stream comprising predominantly ethylbenzene, a stream comprising predominantly styrene and a stream of heavy compounds,
in which the hydrogen partial pressure and the residence time of the gas phase are controlled in the pyrolysis section in step d), and in which the hydrogen partial pressure in the pyrolysis section in step d) is controlled by the presence of a hydrogen donor in the polystyrene feedstock and/or in the supply to the pyrolysis section in step d) and/or by supplying an inert gas to the pyrolysis section in step d).

2. Depolymerization process according to the preceding claim, wherein the hydrogen partial pressure in the pyrolysis section in step d) is controlled by the presence of a hydrogen donor selected from polyolefins, paraffin waxes and mixtures thereof in the polystyrene feedstock and/or in the stream rich in oligomers from step c).

3. Depolymerization process according to the preceding claim, wherein the hydrogen partial pressure in the pyrolysis section in step d) is controlled by the presence of a hydrogen donor in the styrene feedstock, the hydrogen donor being polypropylene and the polypropylene to polystyrene weight ratio in the polystyrene feedstock being adjusted to between 1% by weight and 15% by weight, preferably between 1% and 3% by weight.

4. Depolymerization process according to any one of Claims 1 to 3, wherein the hydrogen partial pressure in the pyrolysis section in step d) is controlled by the presence of a hydrogen donor selected from polyolefins, paraffin waxes, dihydrogen and mixtures thereof in the supply to the pyrolysis section in step d).

5. Depolymerization process according to any one of the preceding claims, wherein the second pyrolysis step d) is operated at a temperature ranging from 400°C to 900°C, preferably from 500°C to 800°C, more preferably between 650°C and 800°C, and at a pressure ranging from 1.0 bar to 7.5 bar, preferably ranging from 3 bar to 6 bar and more preferably ranging from 3 bar to 4.5 bar.

6. Depolymerization process according to any one of the preceding claims, wherein the second pyrolysis step d) is operated with a gas-phase residence time ranging from 10 s to 30 s, preferably ranging from 10 s to 25 s and very preferably ranging from 15 s to 20 s.

7. Depolymerization process according to the preceding claim, wherein the residence time of the gas phase is controlled by supplying an inert gas to the pyrolysis section in step d), preferably an inert gas selected from nitrogen, argon, helium, neon, xenon and krypton, preferably nitrogen.

8. Depolymerization process according to any one of the preceding claims, wherein the first pyrolysis step b) and/or the second pyrolysis step d) employs a microwave pyrolysis section.

9. Depolymerization process according to any one of the preceding claims, wherein the separation step c) employs a distillation column supplied at the bottom of the column with the first gaseous pyrolysis stream from step b) and the second gaseous pyrolysis stream from step d) and producing at the head of the column a stream rich in light compounds and at the bottom a stream rich in oligomers and, through a sidestream withdrawal, a stream rich in aromatics, said column having as sole heat input said first gaseous pyrolysis stream from step b) and said second gaseous pyrolysis stream from step d).

10. Depolymerization process according to any one of the preceding claims, wherein step e) of separating the stream rich in aromatics includes a first separation section supplied with the stream rich in aromatics from step c), which makes it possible to separate a stream comprising predominantly ethylbenzene and a styrene raffinate, and a second separation section supplied with the styrene raffinate from the first separation section and producing a stream comprising predominantly styrene and a stream of heavy compounds.

11. Depolymerization process according to any one of the preceding claims, wherein step e) of separating the stream rich in aromatics is carried out in a divided-wall distillation column.

12. Depolymerization process according to any one of the preceding claims, wherein the stream comprising predominantly ethylbenzene from step e) is separated into at least a stream comprising predominantly toluene, a stream that comprises ethylbenzene and a stream that comprises styrene.

13. Depolymerization process according to any one of the preceding claims, wherein the stream of heavy compounds from step e) is separated into at least a stream comprising styrene, a stream that comprises AMS and a stream that comprises oligomers.
